# EUROPEAN PATENT SPECIFICATION

(11) **EP 2 727 994 B1**
(45) Date of publication and mention of the grant of the patent: **10.05.2017**
(21) Application number: 12803868.4
(22) Date of filing: 19.03.2012
(51) Int. Cl.: A23K 10/00, A23K 20/00, A23K 50/00, A23K 30/15, C12N 1/20, C12R 1/46, C07K 14/315, A23K 10/12, A23K 50/10, A23K 30/18

(54) **NOVEL LACTIC ACID BACTERIUM AND METHOD FOR PREPARING SILAGE OR FERMENTED FEED USING SAME**
NEUES MILCHSÄUREBAKTERIUM UND VERFAHREN ZUR HERSTELLUNG EINER SILAGE ODER EINES FERMENTIERTEN FUTTERS DAMIT
BACTÉRIE LACTIQUE INÉDITE ET PROCÉDÉ DE PRÉPARATION DE PRODUITS D'ENSILAGE OU D'ALIMENTS FERMENTÉS L'UTILISANT

(30) Priority: 29.06.2011 JP 2011144726
(43) Date of publication of application: 07.05.2014
(73) Proprietor: Snow Brand Seed Co., Ltd., Hokkaido 004-8531 (JP)
(72) Inventor: KITAMURA, Toru, Ebetsu-shi Hokkaido 069-0832 (JP); OBUCHI, Shun, Kawagoe-shi Saitama 3501165 (JP); HONMA, Mitsuru, Ebetsu-shi Hokkaido (JP); UENISHI, Hiroshi, Kawagoe-shi Saitama 350-1165 (JP); SOEJIMA, Hiroshi, Ebetsu-shi Hokkaido 069-0832 (JP)
(74) Representative: Hartz, Nikolai
(86) International application number: PCT/JP2012/056991
(87) International publication number: WO 2013/001862

(56) References cited:
- EP-A1- 1 273 237
- EP-A2- 0 906 952
- WO-A1-2006/111561
- JP-A- 2001 245 660
- JP-A- 2004 041 064
- JP-A- 2008 048 683
- AKIRA KITAMURA: 'Silage-yo Nyusankin towa' DAIRY JAPAN January 2012, pages 66 - 69, XP008173044
- AKIRA KITAMURA: 'Rakusan Hakko ni yoru Henpai Kaizen o Mezasu Atarashi Silage-yo Nyusankin 'Si-Master'' DAIRYMAN vol. 61, no. 12, 01 December 2011, page 70, 71, XP008172349

## Description

### Technical Field

The present invention relates to a lactic acid bacterium that can suppress butyric acid fermentation, to a microbial additive containing the bacterium, and to a method for preparing silage or a fermented feed, the method employing the microbial additive.

### Background Art

Silage, which is produced through lactic acid fermentation of livestock's basic feed such as grass and forage crops under anaerobic conditions, is now essential feed used in dairy farming. Thus, preparation of high-quality silage is a very important technique for dairy farmers. In the course of lactic acid fermentation, unfavorable fermentation often occurs in parallel. This problematic fermentation is caused by more active proliferation of butyric acid bacteria compared with lactic acid bacteria in silage, resulting in an increase in the butyric acid content of the silage. When the butyric acid content of silage increases, feed palatability and feed intake by cows decrease, and problematic diseases such as ketosis are triggered. In order to prevent such spoilage caused by butyric acid bacteria, as a generally employed technique, a lactic acid bacterium is used in silage additive. In fact, a variety of lactic acid bacteria have been used, and most of them suppress butyric acid fermentation by accelerating lactic acid fermentation to lower pH. However, under butyric acid fermentation-promoting conditions (particularly high water content, low sugar content, and contamination of a large amount of soil), these lactic acid bacteria may fail to sufficiently inhibit butyric acid bacteria. Even at present, unavoidable production of silage spoiled through butyric acid fermentation is a problem.

In order to attain the effect of a lactic acid bacterium added to silage, raw grass must have a soluble saccharide content (dry matter) of 5.0 to 6.0% (equivalent to 1.0 to 3.0% in fresh matter) or higher (Non-Patent Document 1). Also, since many types of grass and forage crops have low sugar content, lactic acid bacteria which had ever been reported often failed to exhibit sufficient effect on silage and to suppress butyric acid fermentation.

Meanwhile, some lactic acid bacteria are known to produce a bacteriocin, an antibacterial protein, through fermentation or culturing. Among such bacteriocins, nisin is an antibacterial protein which is formed of 34 amino acids produced by some strains of *Lactococcus lactis,* lactic acid bacteria, and contains lanthionine, 3-methyllanthionine, dehydroalanine, dehydrbutyrine, and 2-aminobutyric acid in the molecule thereof. Nisin has several analogues, and structures of three nisin analogues; nisin A, nisin Z, and nisin Q, have been already determined. In recent years, nisin U, produced by *Streptococcus uberis,* and nisin F, produced by *Lactococcus lactis,* have been reported.

Nisin has a wide antibacterial spectrum with respect to Gram-positive bacteria including a butyric acid bacterium. Thus, nisin may inhibit a butyric acid bacterium when added to silage. Actually, nisin A, which is a nisin analogue, has been widely used in the food field as a food preservative for cheese and canned vegetables, since nisin A has been approved by WHO and FAO. However, in the case where nisin is directly added to food, high-concentration nisin must be produced by culturing a lactic acid bacterium, along with extraction, purification, and drying steps. Thus, the total process requires high cost. In addition, nisin exhibits an immediate antibacterial effect, but the effect has difficulty in persisting and may be reduced in some cases through interaction with the ingredient to which nisin has been added.

Under such circumstances, studies have been conducted on addition of a nisin-producing lactic acid bacterium to foods such as cheese, sauerkraut, soybean *miso,* and rice *miso* (Non-Patent Documents 2 to 5), but such a lactic acid bacterium has never been applied in practice to grass and forage crops (raw material of silage), food production byproducts, etc. One conceivable reason therefor is as follows. These silage raw materials are poor in nutrients including sugar that is an essential ingredient for the growth of a lactic acid bacterium as compared with food materials. Also, since these silage raw materials are not subjected to a sterilization step, which is generally performed for food materials, a lactic acid bacterium grows competitively with other microorganisms. Thus, even though a nisin-producing lactic acid bacterium, which is effective for food materials, is added, the effect may fail to be fully attained. To overcome this drawback, there have been reported a lactic acid bacterium that produces nisin at a high concentration in a synthetic culture medium, and production of nisin in a fermented barley extract medium (Patent Document 1 and Non-Patent Document 6). However, no prior art document discloses production of nisin at a high concentration in a silage material containing a different nutrient, although production of nisin at a high concentration in a synthetic culture medium or a fermented barley extract medium is disclosed.

In the case where a lactic acid bacterium is added to silage, *Lactococcus lactis* RO50, being capable of producing bacteriocin, is known to be added to silage raw material (Patent Document 2). However, sufficient fermentation-improving affect has not been attained in some cases, indicating that the effect of *Lactococcus lactis* RO50 is not consistently attained. Furthermore, whether or not *Lactococcus lactis* RO50 produces bacteriocin in silage has not been clearly elucidated (Non-Patent Documents 7 and 8). Patent Document 3 discloses a silage additive method using a nisin-producing lactic acid bacterium and a nisin-resistant lactic acid bacterium, and a nisin activity of 200 IU/g detected in silage. However, as compared with the case where a nisin-non-producing lactic acid bacterium is added, fermentation quality, including pH, total organic acid content, ratio of ammonium nitrogen to total nitrogen etc., were not significantly changed. In addition, the experiment was performed at a sugar content where a generally employed lactic acid bacterium can result in favorable fermentation (5.0% or higher (dry matter), 1.0% or higher (fresh matter)). Thus, Patent Document 3 does not disclose the effect of the lactic acid bacterium under low-sugar-content severe conditions.

### Citation List

### Patent Documents

Patent Document 1: WO2005/080550
Patent Document 2: JP-A-2004-41064
Patent Document 3: EP1273237 A1

### Non-Patent Documents

Non-Patent Document 1: Takayoshi MASUKO (1999), Progress in Silage Science, Dairy Japan Corporation, 99-101
Non-Patent Document 2: Delves-Broughton, J. et al., Antonie van Leeuwenhoek (1996) 69: 193-202
Non-Patent Document 3: Harris, L. J. et al., Appl. Environ. Microbiol.(1992) 58: 1484-1589
Non-Patent Document 4: Takeo Kato et al., Biosci. Biotechnol. Biochem. (1999) 63: 642-647
Non-Patent Document 5: Takeo Kato et al., Biosci. Biotechnol. Biochem. (2001) 65: 330-337
Non-Patent Document 6: Furuta Y. et al., J. Biosci. Bioeng. (2008) 106: 393-397
Non-Patent Document 7: Cai Yimin et al., Research Results of NARO Institute of Livestock and Grassland Science (2002) No. 2: 53-54
Non-Patent Document 8: Hisami Kobayashi et al., Japanese Journal of Grassland Science (2010) 56: 39-46

### Summary of the Invention

### Problems to be Solved by the Invention

An object of the present invention is to selectively obtain a lactic acid bacterium that can produce nisin at a high concentration even under silage fermentation conditions where butyric acid fermentation readily proceeds, to thereby provide a technique for consistently preparing high-quality silage and a high-quality fermented feed, the technique including adding to silage a microbial additive containing the lactic acid bacterium having such a capacity.

### Means for Solving the Problems

In order to select a lactic acid bacterium which can exert excellent performance in silage, the present inventors have prepared a grass broth medium to which no sugar had been added and which has considerably low mono- and disaccharide contents. The inventors have cultured lactic acid bacteria by use of the thus-prepared medium, and selected nisin-producing lactic acid bacteria through screening. As a result, the inventors have successfully obtained through screening a lactic acid bacterium having higher nisin activity in the grass broth medium, as compared with known nisin-producing lactic acid bacterium strains. The present invention has been accomplished on the basis of this finding.

Accordingly, the present invention is directed to the following [1] to [5].
[1] A lactic acid bacterium that produces nisin in a grass broth medium having a mono- and disaccharide total content of 0.1 to 0.4 mass% at a concentration of 40 IU or more per mL of a supernatant of the medium, wherein the lactic acid bacterium is a *Lactococcus lactis SBS-0001* strain (*NITE BP-1107*).
[2] A microbial additive for preparing silage, which additive contains a lactic acid bacterium that produces nisin in a grass broth medium having a mono- and disaccharide total content of 0.1 to 0.4 mass% at a concentration of 40 IU or more per mL of a supernatant of the medium, wherein the lactic acid bacterium is *a Lactococcus lactis SBS-0001* strain (*NITE BP-1107*).
[3] The microbial additive for preparing silage according to claim 2, which additive further contains a lactic acid bacterium having acid tolerance, wherein the lactic acid bacterium having acid tolerance is a *Lactobacillus paracasei SBS-0003* strain (*NITE BP-1109*).
[4] A method for preparing silage or a fermented feed, the method comprising employing a microbial additive for preparing silage containing a lactic acid bacterium that produces nisin in a grass broth medium having a mono- and disaccharide total content of 0.1 to 0.4 mass% at a concentration of 40 IU or more per mL of a supernatant of the medium, wherein the lactic acid bacterium is a *Lactococcus lactis SBS-0001* strain (*NITE BP-1107*).
[5] The method for preparing silage or a fermented feed according to claim 4, wherein the microbial additive for preparing silage further contains a lactic acid bacterium having acid tolerance, wherein the lactic acid bacterium having acid tolerance is a *Lactobacillus paracasei SBS-0003* strain (*NITE BP-1109*).

### Effects of the Invention

Currently, many dairy farmers suffer from butyric acid fermentation of silage and fermented feed. The present invention enables provision of a lactic acid bacterium that produces nisin even in a low-sugar-content material, which is similar to silage to be fermented. The present invention can also provide a microbial additive containing the aforementioned lactic acid bacterium *Lactococcus lactis SBS-0001* strain (*NITE BP-1107*) and an additional lactic acid bacterium *Lactobacillus paracasei SBS-0003* strain (*NITE BP-1109*) having acid tolerance, which additive suppresses butyric acid fermentation, to thereby realize favorable lactic acid fermentation. According to the present invention, silage or a fermented feed which ensures high safety and high feed palatability by livestock can be produced.

### Modes for Carrying Out the Invention

The nisin-producing lactic acid bacterium of the present disclosure is produced through selection of a lactic acid bacterium that produces nisin in a grass broth medium having a mono- and disaccharide total content of 1.0 mass% or less, preferably 0.7 mass% or less, more preferably 0.4 mass% or less.

In the present disclosure, no particular limitation is imposed on the grass for producing a grass broth medium in which selection of the nisin-producing lactic acid bacterium is performed, so long as the grass is used as silage or fermented feed. Examples of the grass include alfalfa, clover, timothy, orchardgrass, reed canarygrass, quackgrass, Italian ryegrass, perennial ryegrass, tall fescue, meadow fescue, festulolium, Kentucky bluegrass, redtop, Guineagrass, Rhodesgrass, and napiergrass. In consideration of conditions where butyric acid fermentation is likely to proceed, the grass is preferably a low-sugar-content grass such as alfalfa, clover, timothy, orchardgrass, reed canarygrass, quackgrass, Guineagrass, Rhodesgrass, or napiergrass. The grass may be as-mown green forage or a chilled or frozen product thereof. Alternatively, a dry powder thereof produced through blow-drying or lyophilizing raw grass followed by pulverizing may also be used. Still alternatively, generally available feed products such as lucerne pellets and hay cubes may be used.

The grass broth medium used in the present disclosure is a medium containing, as a sole nutrient, a liquid obtained by immersing the above grass in hot water. The temperature of the hot water is preferably 50 to 100°C, more preferably 70 to 100°C. The time for immersing the grass in hot water is preferably 10 to 180 minutes, more preferably 30 to 120 minutes. If required, the grass infusion liquid is preferably filtered to remove solid matter before use. In order to select a lactic acid bacterium that produces nisin under low-sugar-content conditions, the grass broth medium used in the disclosure is preferably diluted with water so that the mono- and disaccharide total content thereof is 1.0 mass% or less, preferably 0.7 mass% or less, more preferably 0.4 mass% or less. The lower limit of the mono- and disaccharide total content is preferably 0.1 mass%, more preferably 0.2 mass%.

In selection of the nisin-producing lactic acid bacterium of the present invention, culture conditions under which conventional lactic acid bacteria can be grown may be employed. In one example, lactic acid bacteria are inoculated to a grass broth medium at a cell concentration of 0.1 to 1.0 mass% and static culturing is performed at 25 to 35°C for 8 to 24 hours.

Needless to say, since a nisin-producing strain has tolerance to nisin, strains which can grow in a nisin-added medium can be selected in primary screening. In this case, the medium for screening may be an MRS medium containing nisin at a concentration of 100 to 1,000 IU/mL. A nisin-producing bacterium attaining the object of the present disclosure can be selected through measuring the nisin concentration or a nisin activity of a culture supernatant. The nisin concentration may be determined through a technique such as high-performance liquid chromatography. The nisin activity may be determined as the antibacterial activity with respect to a nisin-sensitive strain of a lactic acid bacterium or a butyric acid bacterium; for example, *Lactobacillus bulgaricus.* The nisin activity is preferably determined as the nisin content. The nisin content of a culture supernatant may be determined through a generally known method. In one specific procedure, the nisin activity may be determined through the agar well method. More specifically, penicillin cups (diameter: 8 mm) are placed on a 1.5% agar MRS medium plate. On the agar medium, a 0.75% agar MRS medium containing 0.1% culture solution of *Lactobacillus bulgaricus* JCM1002^{T} as an indicator bacterium is stacked. After solidification of agar, the penicillin cups are removed, and the plate is employed as a medium plate for an antibacterial test. Each culture supernatant is filtered for eliminating unnecessary microorganisms and added to wells formed by means of the penicillin cups. The plate is anaerobically cultured at 37°C for 24 hours, and the diameter of an inhibition ring with respect to the indicator bacterium is measured. When a nisin preparation (product of Sigma, nisin content: 2.5%) is employed as a standard, the nisin activity per microgram of nisin can be obtained as 40 IU (1 IU being equivalent to 1 µg of nisin preparation).

The lactic acid bacterium *Lactococcus lactis SBS-0001* strain (*NITE BP-1107*) produces nisin in a grass broth medium having a mono- and disaccharide total content of 0.1 to 0.4 mass% at a concentration of 40 IU or more per mL of a supernatant of the medium.

In one preferred embodiment of the present invention, *Lactococcus lactis SBS-0001* strain (*NITE BP-1107*) produces nisin in a grass broth medium having a mono- and disaccharide total content of 0.1 to 0.7 mass% at a concentration of 100 IU or more per mL of a supernatant of the medium. More preferably, *Lactococcus lactis SBS-0001* strain (*NITE BP-1107*) produces nisin in a grass broth medium having a mono- and disaccharide total content of 0.1 to 0.4 mass% at a concentration of 100 IU or more per mL of a supernatant of the medium.

The aforementioned mono- and disaccharide total content of the grass broth medium is more preferably 0.2 to 0.4 mass%. The nisin production in the medium is 40 IU or more per mL of a supernatant, preferably 100 IU or more, even more preferably 100 to 400 IU, even more preferably 100 to 300 IU.

In addition, even under low pH conditions (i.e., pH of 5), *Lactococcus lactis SBS-0001* strain (*NITE BP-1107*) produces nisin in a grass broth medium having a mono- and disaccharide total content of 0.1 to 0.4 mass% at a concentration of 40 IU or more per mL of a supernatant of the medium. Since *Lactococcus lactis SBS-000*1 strain (*NITE BP-1107*) produces nisin under low pH conditions, the lactic acid bacterium of the present invention exhibits nisin productivity for a long period of time.

The aforementioned nisin-producing lactic acid bacterium is *Lactococcus lactis SBS-0001* strain (*NITE BP-1107*)*.* Other examples include *Lactococcus lactis* SBS-0002 strain (NITE BP-1108), *Lactococcus lactis* SBS-0004 strain, and *Lactococcus lactis* SBS-0005 strain. Of these, *Lactococcus lactis* SBS-0001 strain (NITE BP-1107) and *Lactococcus lactis* SBS-0002 strain (NITE BP-1108) are preferred.

The aforementioned SBS-0001 strain and SBS-0002 strain were deposited to the National Institute of Technology and Evaluation, Patent Microorganisms Depositary (2-5-8, Kazusakamatari, Kisarazu, Chiba, 292-0818, Japan) on June 15, 2011.

The lactic acid bacterium of the present invention can grow even in the presence of low-sugar-content grass and can exhibit fermentation capacity, to thereby mass-produce nisin. Thus, the bacterium of the present invention is useful as an ingredient of a microbial additive for preparing silage.

The aforementioned lactic acid bacterium may be cultured in a conventional culture medium for lactic acid bacteria. No particular limitation is imposed on the medium, so long as the medium can be used for culturing lactic acid bacteria. Examples of the medium which may be used in the invention include a GYP medium (Michio KOZAKI et al., Manual for Experiments of lactobacillus, 1992, Asakura Publishing Co., Ltd.) and an MRS medium (de Man J. C. et al., Journal of Applied Bacteriology, Vol. 23, 130-135 (1960)). No particular limitation is imposed on the culture conditions. Generally, the culturing may be performed at a pH of 5.0 to 7.0 and a temperature of 25 to 40°C for 10 to 24 hours. The lactic acid bacterium culture product may be added to silage or a fermented feed as a concentrate of culture solution or pellets. The concentrate may be frozen for use. Alternatively, the lactic acid bacterium culture product may be lyophilized, spray-dried, or fluidized-bed-dried with an appropriate protective agent or a carrier, and the thus-obtained powder may be used.

The microbial additive of the present invention contains nisin-producing lactic acid bacterium *Lactococcus lactis SBS-0001* strain (*NITE BP-1107*) singly or in combination of a plurality of species. The microbial additive may further contain an additional lactic acid bacterium. Particularly when the additive contains an acid-tolerant lactic acid bacterium, butyric acid fermentation is suppressed, and favorable lactic acid fermentation can be realized. In this case, needless to say, the additional lactic acid bacterium is preferably a strain having high nisin resistance. No particular limitation is imposed on the acid-tolerant lactic acid bacterium, and examples thereof include *Lactobacillus plantarum, Lactobacillus casei, Lactobacillus rhamnosus, Lactobacillus salivarius, Enterococcus faecium, Pediococcus acidilactici,* and *Pediococcus pentosaceus.* Examples of preferred acid-tolerant lactic acid bacteria include *Lactobacillus paracasei* SBS-0003 strain (NITE BP-1109), *Lactobacillus rhamnosus* SBT-2300 strain, and *Lactobacillus plantarum* Chikuso-1 strain. Of these, *Lactobacillus paracasei* SBS-0003 strain (NITE BP-1109) is more preferred.

The aforementioned SBS-0003 strain was deposited to the National Institute of Technology and Evaluation, Patent Microorganisms Depositary (2-5-8, Kazusakamatari, Kisarazu, Chiba, 292-0818, Japan) on June 15, 2011.

In the microbial additive, no particular limitation is imposed on the ratio of the lactic acid bacterium of the present invention to the acid-tolerant lactic acid bacterium. The ratio by mass is preferably 1:100 to 100:1, more preferably 1:9 to 9:1, even more preferably 3:7 to 7:3.

The microbial additive of the present invention may further contain an enzyme in accordance with needs. Examples of the enzyme include cellulase and hemi-cellulase.

The microbial additive of the present invention may be used for preparing a variety of silage and fermented feed products. No particular limitation is imposed on the silage raw material and fermented feed raw material for preparing a variety of silage and fermented feed products, so long as they are generally used as feed. Examples of the grass and forage crop include alfalfa, clover, timothy, orchardgrass, reed canarygrass, quackgrass, Italian ryegrass, perennial ryegrass, tall fescue, meadow fescue, festulolium, Kentucky bluegrass, redtop, Guineagrass, Rhodesgrass, napiergrass, oat, barley, rye, sorghum, Sudangrass, Japanese millet, corn, and feed rice. Examples of the food production by-product include brewer's grain, *Tofu* (soybean curd) residue, tea residue, *Shochu* (Japanese sprits) residue, whisky residue, beet pulp, bagasse, coffee residue, juice residue, kale residue, and starch residue. Examples of the agricultural by-product include rice straw, straw, and sub-standard vegetables. The microbial additive of the present invention may be added to a fermented TMR, which is prepared by mixing a food production by-product, silage, an agricultural by-product, hay, concentrated feed, vitamins and minerals, etc. These silage raw materials and fermented feed raw materials are preferably used after the water content thereof is adjusted to 40 to 90 mass%.

Examples of the method of adding the microbial additive include dissolving or suspending the additive in water and spraying the liquid to the raw material, and spreading or mixing the microbial additive in the powder form to or with the raw material.

The number of cells added to the raw material is preferably 10³ to 10⁷ cells in total per gram of raw material, more preferably 10⁴ to 10⁶ cells.

In preparation of silage or fermented feed, raw materials are generally fermented under anaerobic conditions. No particular limitation is imposed on the silo for fermentation, so long as the anaerobic state can be maintained to some extent. Examples of the silo include a bunker silo, a stack silo, a trench silo, a tower silo, an underground silo, a block silo, a cup silo, a roll bale, and a flexible container bag. Generally, fermentation is performed at an ambient outside temperature (about 5 to about 30°C) for one week or longer.

In the silage and fermented feed produced by use of the microbial additive of the present invention, butyric acid fermentation is effectively suppressed. Thus, the silage and fermented feed provide excellent feed palatability and feed intake by cows and cause no ketosis.

### Examples

The present invention will next be described in detail by way of examples, which should not be construed as limiting the invention thereto.

### <Example 1> Nisin activity in grass broth medium

Each lactic acid bacterium was cultured in a grass broth medium, and the nisin activity of a supernatant of the culture solution was measured.

The grass broth medium was prepared in the following manner. Distilled water (1 L) was added to lucerne pellets (100 g), and the mixture was heated in a boiling water bath to a liquid temperature of 90°C. Thereafter, grass ingredients were extracted at 90°C for 30 minutes. The mixture was filtered with gauze, and the thus-separated residue was squeezed. The filtrate was centrifuged, and the liquid was filtered with filter paper. The thus-obtained filtrate was diluted to 1 L, and the dilute was dispensed to test tubes. These samples were sterilized in an autoclave (115°C, 20 minutes).

The sugar content of the grass broth medium was determined in the following manner. Acetonitrile (840 µL) was added to the medium (360 µL), followed by mixing, and the mixture was allowed to stand at room temperature for 15 minutes. The mixture was centrifuged (15,000 rpm, 15 minutes), to thereby remove precipitates, and the supernatant was filtered with a 0.2-µm filter. The filtrate was analyzed through HPLC. The amounts of monosaccharides and disaccharides (xylose, glucose, fructose, and sucrose) were summed, to thereby obtain the sugar content.

Each of the lactic acid bacteria shown in Table 1 was precultured in an MRS liquid medium. The thus-obtained preculture solution was centrifuged, to thereby remove the medium. To the cell pellets, an equiamount of physiological saline was added. The cell suspension was inoculated at 1% to the grass broth medium, and static culturing was performed at 32°C for 8 hours. The absorbance (at 660 nm) of the culture solution was measured. Then, the culture solution was centrifuged and filtered with a 0.2-µm filter, to thereby remove cells. The nisin activity of the culture supernatant was determined through the agar well method.

**[Table 1]**

| Bacterial strain | |
|---|---|
| SBS-0001 | Test strain |
| SBS-0002 | Test strain |
| RO50 | Control strain: bacteriocin-producing |
| ATCC 11454 | Control strain: nisin A-producing |
| JCM 7638 | Control strain: nisin Z-producing |

The agar well method was carried out in the following manner. Specifically, penicillin cups (diameter: 8 mm) were placed on a 1.5% agar MRS medium plate. On the agar medium, a 0.75% agar MRS medium containing 0.1% culture solution of *Lactobacillus bulgaricus* JCM1002^{T} as an indicator bacterium was stacked. After solidification of agar, the penicillin cups were removed, and the plate was employed as a medium plate for an antibacterial test. Each culture supernatant was filtered for eliminating unnecessary microorganisms and added to wells formed by means of the penicillin cups. The plate was anaerobically cultured at 37°C for 24 hours, and the diameter of an inhibition ring with respect to the indicator bacterium was measured. A nisin additive (product of Sigma, nisin content: 2.5%) was employed as a standard, and the nisin activity per microgram of nisin was employed as 40 IU (1 IU being equivalent to 1 µg of nisin additive).

**[Table 2]**

| Lactobacillus strain | O. D. 660 | Nisin activity (IU/mL) |
|---|---|---|
| SBS-0001 (Ex.) | 0.88 | 165.56 |
| SBS-0002 (Ex.) | 0.85 | 165.56 |
| RO50 (Control) | 0.82 | 30.36 |
| ATCC 11454 (Control) | 0.75 | 0 |

| | | |
|---|---|---|
| Sugar content of grass broth medium: 0.33% | | |

**[Table 3]**

| Lactobacillus strain | O. D. 660 | Nisin activity (IU/mL) |
|---|---|---|
| SBS-0001 (Ex.) | 0.75 | 145.03 |
| SBS-0002 (Ex.) | 0.84 | 169.94 |
| JCM 7638 (Control) | 0.77 | 0 |

| | | |
|---|---|---|
| Sugar content of grass broth medium: 0.31% | | |

**[Table 4]**

| Lactobacillus strain | O. D. 660 | Nisin activity (IU/mL) |
|---|---|---|
| SBS-0001 (Ex.) | 1.15 | 192.91 |
| SBS-0002 (Ex.) | 1.21 | 229.66 |
| JCM 7638 (Control) | 1.14 | 96.05 |

| | | |
|---|---|---|
| Sugar content of grass broth medium: 0.68% | | |

**[Table 5]**

| Lactobacillus strain | O. D. 660 | Nisin activity (IU/mL) |
|---|---|---|
| SBS-0001 (Ex.) | 0.53 | 44.59 |
| SBS-0002 (Ex.) | 0.54 | 40.25 |
| RO50 (Control) | 0.62 | 0 |

| | | |
|---|---|---|
| Sugar content of grass broth medium: 0.33% Initial pH: 5.0 | | |

As is clear from Tables 2 and 3, in a grass broth medium having a sugar content of about 0.3%, nisin A-producing ATCC 11454 strain, nisin Z-producing JCM 7638 strain, and bacteriocin-producing RO50 strain exhibited the same growth score (O. D. 660) as that of SBS-0001 strain and SBS-0002 strain, but exhibited very low nisin activity. In contrast, SBS-0001 strain and SBS-0002 strain exhibited very high nisin activity in the grass broth medium having a sugar content of about 0.3%.

Also, as is clear from Table 4, SBS-0001 strain and SBS-0002 strain exhibited higher growth score and nisin activity in a grass broth medium having a sugar content of about 0.7% than in a grass broth medium having a sugar content of about 0.3%. In contrast, JCM 7638 strain exhibited an activity that is half or lower the activity of SBS-0001 strain or SBS-0002 strain.

The pH of silage is known to lower in the course of fermentation. Thus, in order to ensure a long-term persistent effect, nisin is preferably produced also at low pH. However, nisin productivity is known to drop as the pH in culture lowers. As is clear from Table 5, when the initial pH of the grass broth medium was adjusted to 5.0, all the tested strains exhibited reduced nisin activity in each culture supernatant. Although RO50 strain exhibited a nisin activity of 0, SBS-0001 strain and SBS-0002 strain exhibited a nisin activity of 40 IU/mL or higher.

### <Example 2> Preparation of silage and fermentation quality

Silage samples were prepared by adding a lactic acid bacterium to grass, and the fermentation quality of each sample was investigated.

The lactic acid bacteria shown in Table 6 were prepared in the following manner. Each cell strain was inoculated to a GYP liquid medium, and cultured at 37°C for 24 hours. The culture solution was centrifuged (6,500 rpm, 15 minutes), and the supernatant was removed. To the thus-separated cell pellets, 10% trehalose and 1% sodium glutamate solution were added, to thereby form a suspension. The suspension was dried by means of a freeze-drier.

**[Table 6]**

| Bacterial strain | |
|---|---|
| SBS-0001 | Test strain: *Lactococcus lactis* |
| SBS-0002 | Test strain: *Lactococcus lactis* |
| SBS-0003 | Test strain: *Lactobacillus paracasei* |
| SBT-2300 | Control strain: *Lactobacillus rhamnosus* Strain used in commercial product Snow Lact L (Snow Brand Seed Co., Ltd.) |
| RO50 | Control strain: *Lactococcus lactis* |

The silage samples were prepared in the following manner. Timothy, orchardgrass, reed canarygrass, quackgrass, Italian ryegrass, and rye were mowed in a heading stage, and alfalfa was mowed in a blooming stage. In each case, the mown plant was shredded into pieces of a length of about 2 cm. Freeze-dried powder of each lactic acid bacterium was dissolved in water. When one single strain was added, the bacterium was added in an amount of 1.0 × 10⁵ cfu to 1 g of raw grass material, followed by mixing, whereas when two strains were added, each bacterium was added in an amount of 5.0 × 10⁴ cfu to 1 g of raw grass material, followed by mixing. Each raw grass material was placed in a plastic bottle made of polyethylene having a volume capacity of 1 L or a plastic pouch (nylon-polyethylene laminate film). In the case of a plastic bottle, raw grass material (600 g) was forcedly placed into the bottle, and the bottle was sealed with a lid. To a plastic pouch, raw grass material (100 g) was added, and the pouch was evacuated by means of a commercial suction pump for sealing. These samples were subjected to fermentation at 25°C for 2 months. The thus-obtained silage samples were analyzed in terms of pH (by a pH meter) and organic acid content (through HPLC).

**[Table 7]**

| Fermentation quality of timothy silage (plastic bottle) | | | | |
|---|---|---|---|---|
| Treatment | pH | Organic acid content (% to fresh matter) | | |
| | | Lactic acid | Acetic acid | Butyric acid |
| No addition (Control) | 4.82 | 0 | 0.87 | 0.74 |
| SBT-2300 (control) | 4.81 | 0 | 0.79 | 0.78 |
| SBT-2300 + RO50 (Control) | 4.86 | 0 | 0.38 | 0.86 |
| SBT-2300 + SBS-0001 (Ex.) | 4.56 | 0.62 | 0.18 | 0.46 |

**[Table 8]**

| Fermentation quality of reed canarygrass silage (plastic bottle) | | | | |
|---|---|---|---|---|
| Treatment | pH | Organic acid content (% to fresh matter) | | |
| | | Lactic acid | Acetic acid | Butyric acid |
| No addition (Control) | 5.35 | 0 | 0.80 | 0.92 |
| SBT-2300 (Control) | 4.92 | 0.05 | 0.25 | 1.08 |
| SBT-2300 + RO50 (Control) | 4.45 | 0.72 | 0.05 | 0.55 |
| SBT-2300 + SBS-0001 (Ex.) | 4.30 | 0.96 | 0.06 | 0.38 |

**[Table 9]**

| Fermentation quality of timothy silage (pouch) | | | | |
|---|---|---|---|---|
| Treatment | pH | Organic acid content (% to fresh matter) | | |
| | | Lactic acid | Acetic acid | Butyric acid |
| No addition (Control) | 5.10 | 0 | 0.22 | 0.77 |
| SBT-2300 (Control) | 4.25 | 0.87 | 0.15 | 0.51 |
| SBS-0003 (Control) | 3.97 | 1.43 | 0.30 | 0.11 |
| SBS-0003 + SBS-0001 (Ex.) | 3.80 | 1.71 | 0.28 | 0.01 |
| SBS-0003 + SBS-0002 (Ex.) | 3.81 | 1.79 | 0.30 | 0 |

**[Table 10]**

| Fermentation quality of orchardgrass silage (pouch) | | | | |
|---|---|---|---|---|
| Treatment | pH | Organic acid content (% to fresh matter) | | |
| | | Lactic acid | Acetic acid | Butyric acid |
| No addition (Control) | 5.11 | 0 | 0.57 | 0.94 |
| SBT-2300 (Control) | 5.02 | 0.02 | 0.49 | 0.89 |
| SBS-0003 (Control) | 4.73 | 0.25 | 0.30 | 0.81 |
| SBS-0003 + SBS-0001 (Ex.) | 3.82 | 2.61 | 0.18 | 0 |
| SBS-0003 + SBS-0002 (Ex.) | 3.80 | 2.22 | 0.15 | 0 |

**[Table 11]**

| Fermentation quality of alfalfa silage (pouch) | | | | |
|---|---|---|---|---|
| Treatment | pH | Organic acid content (% to fresh matter) | | |
| | | Lactic acid | Acetic acid | Butyric acid |
| No addition (Control) | 5.63 | 0 | 0.74 | 1.08 |
| SBT-2300 (Control) | 5.64 | 0 | 0.82 | 1.18 |
| SBS-0003 (Control) | 5.69 | 0 | 0.84 | 1.09 |
| SBS-0003 + SBS-0001 (Ex.) | 4.85 | 1.31 | 0.88 | 0.15 |
| SBS-0003 + SBS-0002 (Ex.) | 4.74 | 1.43 | 0.89 | 0 |

**[Table 12]**

| Fermentation quality of reed canarygrass silage (plastic bottle) | | | | |
|---|---|---|---|---|
| Treatment | pH | Organic acid content (% to fresh matter) | | |
| | | Lactic acid | Acetic acid | Butyric acid |
| No addition (Control) | 4.98 | 0 | 1.10 | 0.78 |
| SBT-2300 (Control) | 5.11 | 0 | 0.77 | 0.94 |
| SBS-0003 + SBS-0001 (Ex.) | 3.89 | 1.57 | 0.10 | 0 |

**[Table 13]**

| Fermentation quality of quackgrass (plastic bottle) | | | | |
|---|---|---|---|---|
| Treatment | pH | Organic acid content (% to fresh matter) | | |
| | | Lactic acid | Acetic acid | Butyric acid |
| No addition (Control) | 5.47 | 0 | 0.64 | 1.02 |
| SBT-2300 (Control) | 4.22 | 1.49 | 0.10 | 0.44 |
| SBS-0003 + SBS-0001 (Ex.) | 3.80 | 2.59 | 0.18 | 0 |

**[Table 14]**

| Fermentation quality of Italian ryegrass (pouch) | | | | |
|---|---|---|---|---|
| Treatment | pH | Organic acid content (% to fresh matter) | | |
| | | Lactic acid | Acetic acid | Butyric acid |
| No addition (Control) | 5.17 | 0 | 0.74 | 1.40 |
| SBT-2300 (Control) | 4.18 | 2.03 | 0.21 | 0.31 |
| SBS-0003 + SBS-0001 (Ex.) | 3.73 | 3.06 | 0.10 | 0 |
| SBS-0003 + SBS-0002 (Ex.) | 3.76 | 3.05 | 0.12 | 0 |

**[Table 15]**

| Fermentation quality of rye silage (pouch) | | | | |
|---|---|---|---|---|
| Treatment | pH | Organic acid content (% to fresh matter) | | |
| | | Lactic acid | Acetic acid | Butyric acid |
| No addition (Control) | 5.58 | 0.24 | 0.33 | 0.98 |
| SBT-2300 (Control) | 5.17 | 0.71 | 0.29 | 0.63 |
| SBS-0003 + SBS-0001 (Ex.) | 4.16 | 2.03 | 0.10 | 0 |
| SBS-0003 + SBS-0002 (Ex.) | 4.15 | 2.01 | 0.10 | 0 |

As is clear from Tables 7 and 8, when RO50 strain, which can produce nisin in a grass broth medium at 40 IU/mL or less, was added to a material whose fermentation quality cannot virtually be improved by SBT-2300 strain (used as a commercial lactic acid bacterium for silage), the fermentation quality of the silage was not substantially improved, or only a weak butyric acid reduction effect resulted. In contrast, when SBS-0001 strain, exhibiting high nisin productivity in a grass broth medium, was added to a raw grass material, the butyric acid level was clearly reduced, leading to improvement in silage fermentation quality.

As is clear from Table 9, SBS-0003 strain was found to have higher lactic acid fermentation performance than that of SBT-2300 strain. By adding SBT-0001 strain or SBT-0002 strain, the butyric acid level was further reduced.

As is clear from Tables 10 and 11, by adding SBS-0001 strain or SBS-0002 strain in addition to SBS-0003 strain, the butyric acid content of a silage whose fermentation quality cannot be improved by SBT-2300 strain or SBS-0003 strain was considerably reduced, and remarkably improved fermentation quality were attained.

As is clear from Tables 9, 10, 11, 12, 13, 14, and 15, by adding SBS-0001 strain or SBS-0002 strain in addition to SBS-0003 strain, the butyric acid levels of a variety of materials which exhibit poor fermentation quality in the absence of conventional bacteria and whose fermentation quality cannot be fully improved by SBT-2300 strain were considerably reduced, and remarkably improved fermentation quality were attained.

## Claims

1. A lactic acid bacterium that produces nisin in a grass broth medium having a mono- and disaccharide total content of 0.1 to 0.4 mass% at a concentration of 40 IU or more per mL of a supernatant of the medium, wherein the lactic acid bacterium is a *Lactococcus lactis* SBS-0001 strain (NITE BP-1107).

2. A microbial additive for preparing silage, which additive contains a lactic acid bacterium that produces nisin in a grass broth medium having a mono- and disaccharide total content of 0.1 to 0.4 mass% at a concentration of 40 IU or more per mL of a supernatant of the medium, wherein the lactic acid bacterium is a *Lactococcus lactis* SBS-0001 strain (NITE BP-1107).

3. The microbial additive for preparing silage according to claim 2, which additive further contains a lactic acid bacterium having acid tolerance, wherein the lactic acid bacterium having acid tolerance is a *Lactobacillus paracasei* SBS-0003 strain (NITE BP-1109).

4. A method for preparing silage or a fermented feed, the method comprising employing a microbial additive for preparing silage containing a lactic acid bacterium that produces nisin in a grass broth medium having a mono- and disaccharide total content of 0.1 to 0.4 mass% at a concentration of 40 IU or more per mL of a supernatant of the medium, wherein the lactic acid bacterium is a *Lactococcus lactis* SBS-0001 strain (NITE BP-1107).

5. The method for preparing silage or a fermented feed according to claim 4, wherein the microbial additive for preparing silage further contains a lactic acid bacterium having acid tolerance, wherein the lactic acid bacterium having acid tolerance is a *Lactobacillus paracasei* SBS-0003 strain (NITE BP-1109).

## Patentansprüche

1. Milchsäurebakterium, das Nisin in einem Gras-Medium erzeugt, das einen Gesamtgehalt an Mono- und Disaccharid von 0,1 bis 0,4 Masseprozent bei einer Konzentration von 40 IE oder mehr pro ml des Überstands des Mediums aufweist, wobei das Milchsäurebakterium der *Lactococcus lactis* SBS-0001-Stamm (NITE BP-1107) ist.

2. Mikrobielles Additiv zur Herstellung von Silage, welches ein Milchsäurebakterium enthält, das Nisin in einem Gras-Medium erzeugt, das einen Gesamtgehalt an Mono- und Disaccharid von 0,1 bis 0,4 Masseprozent bei einer Konzentration von 40 IE oder mehr pro ml des Überstands des Mediums aufweist, wobei das Milchsäurebakterium der *Lactococcus lactis* SBS-0001-Stamm (NITE BP-1107) ist.

3. Mikrobielles Additiv zur Herstellung von Silage nach Anspruch 2, wobei das Additiv ferner ein Milchsäurebakterium enthält, das Säuretoleranz besitzt, wobei das Milchsäurebakterium mit Säuretoleranz der *Lactobacillus paracasei* SBS-0003-Stamm (NITE BP-1109) ist.

4. Verfahren zur Herstellung von Silage oder eines fermentierten Futtermittels, wobei das Verfahren den Einsatz eines mikrobiellen Additivs zur Herstellung von Silage umfasst, das ein Milchsäurebakterium enthält, das Nisin in einem Gras-Medium erzeugt, das einen Gesamtgehalt an Mono- und Disaccharid von 0,1 bis 0,4 Masseprozent bei einer Konzentration von 40 IE oder mehr pro ml des Überstands des Mediums aufweist, wobei das Milchsäurebakterium der *Lactococcus lactis* SBS-0001-Stamm (NITE BP-1107) ist.

5. Verfahren zur Herstellung von Silage oder eines fermentierten Futtermittels nach Anspruch 4, wobei das mikrobielle Additiv zur Herstellung von Silage ferner ein Milchsäurebakterium enthält, das Säuretoleranz besitzt, wobei das Milchsäurebakterium mit Säuretoleranz der *Lactobacillus paracasei* SBS-0003-Stamm (NITE BP-1109) ist.

## Revendications

1. Ferment lactique qui produit de la nisine dans un milieu liquide d'extraits végétaux présentant une teneur totale en mono- et di-saccharides de 0,1 à 0,4 % en masse à une concentration de 40 Ul ou plus par ml de surnageant du milieu, lequel ferment lactique est une souche de *Lactococcus lactis* SBS-0001 (NITE BP-1107).

2. Additif microbien pour préparer un produit d'ensilage, lequel additif contient un ferment lactique qui produit de la nisine dans un milieu liquide d'extraits végétaux présentant une teneur totale en mono- et di-saccharides de 0,1 à 0,4 % en masse à une concentration de 40 UI ou plus par ml de surnageant du milieu, dans lequel le ferment lactique est une souche de *Lactococcus lactis* SBS-0001 (NITE BP-1107).

3. Additif microbien pour préparer un produit d'ensilage selon la revendication 2, lequel additif contient en outre un ferment lactique présentant une tolérance aux acides, dans lequel le ferment lactique possédant une tolérance aux acides est une souche de *Lactobacillus paracasei* SBS-0003 (NITE BP-1109).

4. Procédé pour préparer un produit d'ensilage ou un aliment pour animaux fermenté, lequel procédé comprend l'emploi d'un additif microbien pour préparer un produit d'ensilage contenant un ferment lactique qui produit de la nisine dans un milieu liquide d'extraits végétaux présentant une teneur totale en mono- et di-saccharides de 0,1 à 0,4 % en masse à une concentration de 40 UI ou plus par ml de surnageant du milieu, dans lequel le ferment lactique est une souche de *Lactococcus lactis* SBS-0001 (NITE BP-1107).

5. Procédé pour préparer un produit d'ensilage ou un aliment pour animaux fermenté, dans lequel l'additif microbien pour préparer un produit d'ensilage contient en outre un ferment lactique présentant une tolérance aux acides, dans lequel le ferment lactique possédant une tolérance aux acides est une souche de *Lactobacillus paracasei* SBS-0003 (NITE BP-1109).
